# EUROPÄISCHE PATENTSCHRIFT

(11) **EP 0 398 099 B1**
(45) Veröffentlichungstag und Bekanntmachung des Hinweises auf die Patenterteilung: **22.09.1993**
(21) Anmeldenummer: 90108497.0
(22) Anmeldetag: 07.05.1990
(51) Int. Cl.: C07C 49/395, C07C 49/523, C07C 45/60

(54) **Verfahren zur Herstellung von Cyclopentanonen**
Process for the production of cyclopentanones
Procédé pour la préparation de cyclopentanones

(30) Priorität: 18.05.1989 DE 3916140; 18.05.1989 DE 3916139; 18.05.1989 DE 3916138
(43) Veröffentlichungstag der Anmeldung: 22.11.1990
(73) Patentinhaber: BASF Aktiengesellschaft, 67063 Ludwigshafen (DE)
(72) Erfinder: Merger, Franz, Dr., D-6710 Frankenthal (DE); Witzel, Tom, Dr., D-6700 Ludwigshafen (DE)

(56) Entgegenhaltungen:
- EP-A- 0 266 687
- US-A- 2 863 923
- US-A- 2 875 249
- US-A- 2 995 607
- US-A- 3 812 190

## Beschreibung

Die vorliegende Erfindung betrifft neue und verbesserte Verfahren zur sauerkatalysierten Herstellung von Cyclopentanonen aus 2-Formyl-3,4-dihydropyranen ggf. aus Acroleinen, sowie neue 2,5-disubstituierte 2-Formylcyclopentanone.

Aus Synthesis (1983), 796-797 ist bekannt, daß man 2-Formylcyclopentanon durch Kondensation von Cyclopentanon mit Ameisensäureester in Gegenwart von Kaliumhydrid herstellen kann. Als Base können z.B. auch Natriummethylat (J. Am. Chem. Soc. 67, 1745-1754 (1945)) oder auch Natrium (J. Am. Chem. Soc. 53, 3160-3164 (1931)) eingesetzt werden.

Aus der US-A-2,875,249 ist bekannt, 2,5-Dimethylcyclopentanon diskontinuierlich durch Umsetzung von 2,5-Dimethyl-2-formyl-3,4-dihydropyran (Dimer-Methacrolein) mit einem Überschuß an Salz- oder Schwefelsäure (20-bis 50%ig in Wasser) bei 110 bis 125°C unter Abdestillieren eines Keton-Wasser-Gemisches herzustellen. Die Ausbeute sinkt von 44 % auf 23 %, wenn weniger als die stöchiometrische Menge Mineralsäure eingesetzt wird.

Aus Bull. Chem. Jpn. 54, 3875 - 3876 (1981) ist die Reaktion von n-Alkanalen mit einem Überschuß Formaldehyd sowie stöchiometrischen Mengen Dimethylammoniumhydrochlorid in Dioxan/Ameisensäure bei 200°C zu maximal 26 % 2,5-Dialkylcyclopentanon bekannt.

2,5-Disubstituierte Cyclopentanone lassen sich weiterhin herstellen durch:
- Cyclisierung von 2,5-disubstituierten Adipinsäurediestern in der Gas-phase an Heterogenkatalysatoren, z. B. 2,5-Dimethylcyclopentanon aus 2,5-Dimethyladipinsäuredimethylester an MnO₂/Al₂O₃ (US-A-2,863,923),
- Formylierung von Dienen unter Metallcarbonyl-Katalyse, z.B. 2,5-Dimethylcyclopentanon aus 1,5-Hexadien mit Co₂(CO)₈ nach US-A-2,995,607 oder mit Ni(CO)₄ nach Tetrahedron Lett. (1968), 1003 - 1006.
- Alkylierung von Cyclopentanon, z. B. mit Dimethylsulfat: man erhält hauptsächlich 2,2-Dimethylcyclopentanon und nur geringe Mengen an 2,5-Dimethylcyclopentanon (Bull. Soc. Chim. Fr. (1957), 1064 - 1069).

Aus US-A-3,812,190 ist bekannt, daß sich das Dimere des unsubstituierten Acroleins (2-Formyl-3,4-dihydropyran) in der Gasphase bei 200 bis 350°C an Dehydrierkatalysatoren (CuO, NiO, CoO, Pd) allein oder auf sauren Trägern wie Silicium- oder Aluminiumoxid zu Cyclopentanon umsetzt; bei einer Reaktionstemperatur von 225°C wird bei einem Umsatz von 70 % eine Selektivität von mindestens 80 % angegeben. Das Verfahren ist jedoch nur mit unbefriedigenden Ausbeuten auf die Dimeren der α-substituierten Acroleine übertragbar. In Versuchen, die angegebenen Katalysatoren wie Pd/Al₂O₃ oder NiO zur Herstellung von z. B. Dimethylcyclopentanon aus 2-Formyl-2,5-dimethyl-3,4-dihydropyran (Dimer-Methacrolein) zu nutzen, wurden unter Bedingungen von US-A-3,812,190 nur Selektivitäten von unter 20 % erreicht. Ferner war aus US-A-2,694,077 und US-A-2,823,211 bekannt, daß Formyldihydropyrane säurekatalysiert (Mineralsäuren, Carbonsäuren) mit Wasser zu einem Gemisch aus Adipindialdehyden und Hydroxitetrahydropyranen bzw. zu Dialkylhydroxycaprolactonen reagieren und sich nach Houben-Weyl, Bd. 6, Teil 4, S. 373 - 374 (1966) mit Alkoholen, wie z.B. Methanol zu 6-Methoxi-2,5-dialkyl-2-dimethoximethyl-tetrahydropyran im Gemisch mit einer Reihe von Nebenprodukten, hierunter auch bicyclischen Derivaten umsetzen lassen.

Nachteile der bisher bekannten Verfahren zur Herstellung 2,5-disubstituierter Cyclopentanone sind niedrige Ausbeuten bei technisch hohem Aufwand bzw. der Einsatz kostspieliger Ausgangsstoffe.

Der vorliegenden Erfindung lag daher die Aufgabe zugrunde, Verfahren zu finden, die es ermöglichen, in einfacher Weise auch substituierte Cyclopentanone und 2-Formylcyclopentanone zugänglich zu machen.

Demgemäß wurden neue und verbesserte Verfahren zur Herstellung von Cyclopentanonen der allgemeinen Formel I 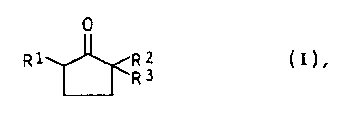
in der R¹ und R² einen organischen Rest oder R¹ oder R² gegebenenfalls Wasserstoff bedeuten und R³ für Wasserstoff oder eine Formylgruppe steht, gefunden, welche dadurch gekennzeichnet sind, daß man 2-Formyl-3,4-dihydropyrane der allgemeinen Formel II 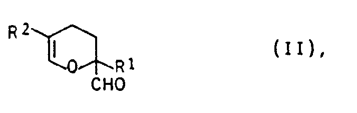
in der R¹ und R² die oben genannte Bedeutung haben,
a) für den Fall, daß R³ für eine Formylgruppe steht, die Umsetzung in Gegenwart eines aciden Heterogenkatalysators bei Temperaturen von 50 bis 500°C,
b) für den Fall, daß R³ für Wasserstoff steht,
   b₁) die Umsetzung mit Wasser oder einem Alkohol in Gegenwart eines aciden Heterogenkatalysators bei Temepraturen von 100 bis 500°C in der Gasphase oder
   b₂) die Umsetzung der Verbindungen II oder Acroleine der allgemeinen Formel III 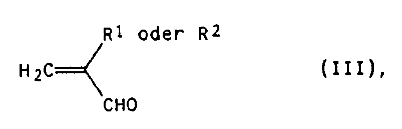 mit Wasser oder mit Wasser in Gegenwart einer Säure oder mit einem Alkohol in Gegenwart einer Säure bei Temperaturen von 150 bis 400°C in der Flüssigphase durchführt, sowie neue 2-Formylcyclopentanone.

### a) Für den Fall, daß R³ für eine Formylgruppe steht sind die 2-Formylcyclopentanone nach folgender Methode erhältlich:

Die Isomerisierung von 2-Formyl-3,4-dihydropyranen II in 2-Formylcyclopentanone I erfolgt durch Kontakt mit aciden Katalysatoren, vorzugsweise aciden Heterogenkatalysatoren im wesentlichen in Abwesenheit von Wasser oder eines Alkohols.

Decarbonylierung der Formylcyclopentanone (R³ = CHO) I zu Cyclopentanonen (R³ = H) I beobachtet man beim erfindungsgemäßen Verfahren nur in unbedeutenden Mengen.

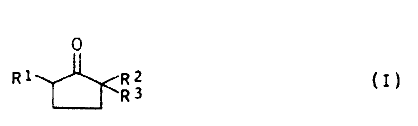

Die Reaktion kann sowohl in der Flüssigphase als auch in der diskontinuierlichen oder vorzugsweise kontinuierlichen Gasphase bei 50 bis 500°C und 0,01 mbar bis 10 bar durchgeführt werden. Die Verwendung eines inerten Lösungsmittels wie z.B. Cyclohexan oder Petrolether kann zweckmäßig sein.

Die Flüssigphasenreaktion kann beispielsweise als Suspensions-, Riesel- oder Sumpfreaktion bei Temperaturen von 50 bis 400°C, vorzugsweise bei Temperaturen von 70 bis 300°C, ggf. unter Druck oder auch bei Unterdruck durchgeführt werden.

Die bevorzugte Gasphasenreaktion kann beispielsweise bei Temperaturen von 100 bis 500°C, vorzugsweise bei 100 bis 400°C und Drücken von 0,1 mbar bis 10 bar, besonders bevorzugt bei 200 bis 300°C und Drücken von 0,5 mbar bis 2 bar durchgeführt werden. Bei der Umsetzung in der Gasphase hält man vorteilhaft eine Katalysatorbelastung von 0,01 bis 40, insbesondere von 0,05 bis 10 g Ausgangsstoff der Formel II je g Katalysator und Stunde ein (WHSV = g Einsatzgemisch/g Katalysator und Stunde). Die Gasphasenreaktion kann in einem Festbett oder in einem Wirbelbett ausgeführt werden.

Das Verfahren wird im allgemeinen bei Normaldruck oder je nach Flüchtigkeit der Ausgangsverbindung bei vermindertem oder erhöhtem Druck vorzugsweise kontinuierlich durchgeführt.

Schwerflüchtige oder feste Ausgangsstoffe werden in gelöster Form z.B. in Tetrahydrofuran-, Toluol- oder Petrolether-Lösung eingesetzt. Allgemein ist auch eine Verdünnung mit diesen Lösungsmitteln oder mit Inertgasen wie N₂, Ar, möglich und zweckmäßig.

Nach der Umsetzung werden die entstandenen Produkte durch übliche Verfahren, z.B. durch Destillation aus dem Reaktionsgemisch isoliert; nichtumgesetzte Ausgangsstoffe werden gegebenenfalls in die Umsetzung zurückgeführt.

Bevorzugt werden Reaktionsprodukte sofort in eine Trennung eingebracht und z.B. in einer Fraktionierkolonne in ihre Einzelkomponenten zerlegt.

Die 2-Formyl-3,4-dihydropyrane II sind durch Erhitzen von α-substituierten Acroleinen III (Houben-Weyl, Bd. VII, Teil 1, S. 130-131; US-A-2,479,283; US-A-2,479,284) nach folgender Reaktionsgleichung erhältlich, wobei R¹ und R² gleich oder verschieden sein können:

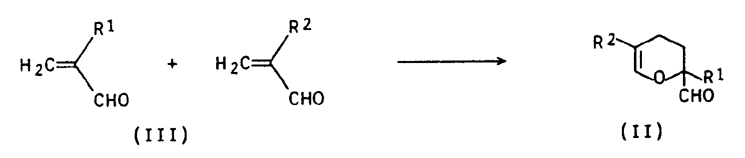

Die Acroleine III können z.B. nach EP-A-58927 durch Kondensation von Alkanalen mit Formaldehyd in hoher Ausbeute hergestellt werden.

Als acide Heterogenkatalysatoren für das erfindungsgemäße Verfahren eignen sich saure Oxide, die im wesentlichen aus Oxiden der II. bis V. Hauptgruppe, der III. bis VII. Nebengruppe, aus Oxiden der Seltenen Erdmetalle oder aus einem Gemisch der genannten Oxide bestehen. So sind beispielsweise Boroxid, Aluminiumoxid, Siliciumoxid, z. B. in Form von Kieselgel, Kieselgur, Quarz oder auch Zinndioxid, Titandioxid, Ceroxid oder ein Gemisch derartiger Oxide geeignet. Zusätzlich können die Katalysatoren noch durch Aufbringen von Zusätzen, wie z. B. Phosphorsäure modifiziert werden. Weitere geeignete Katalysatoren sind Phosphate, wie Aluminium- oder Siliciumaluminiumphosphate. Bevorzugt sind Siliciumoxid enthaltende Katalysatoren. Ganz besonders bewährt hat sich der Einsatz von Siliciumdioxid.

Des weiteren eignen sich Zeolithe.

Die Katalysatoren können wahlweise als 2- bis 4-mm-Stränge oder als Tabletten mit 3 bis 5 mm Durchmesser, als Splitt mit Teilchengrößen von 0,05 bis 1 mm, insbesondere 0,1 bis 0,5 mm, als Pulver mit Teilchengrößen von 0,1 bis 0,5 mm oder als Wirbelkontakt eingesetzt werden.

Die Substituenten R¹ und R² in den Verbindungen I und II haben für das erfindungsgemäße Verfahren folgende Bedeutungen, wobei entweder R¹ oder R² auch für Wasserstoff stehen kann:
- unverzweigtes oder verzweigtes C₁- bis C₁₀-Alkyl, vorzugsweise C₁- bis C₈-Alkyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- unverzweigtes oder verzweigtes C₁- bis C₁₀-Alkyl, das durch cycloaliphatische Reste wie Cycloalkyl, aromatische Reste wie Aryl, besonders Phenyl oder heterocyclische Reste, wie Pyridyl, einen Alkenyl- oder Alkinylrest oder durch Carbalkoxi-, Carboxi-, Alkylamino-, Acyl-, Phosphonester-, Hydroxi-, Ether-, Cycloether-, Thioether-, Cyclothioethergruppen substituiert ist, bevorzugt C₃- bis C₈-Cycloalkyl, C₁- bis C₄-Carbalkoxi, C₂- bis C₄-Alkenyl, besonders bevorzugt C₅- bis C₆-Cycloalkyl und C₁- bis C₇-Carbalkoxi.

Bevorzugt sind Verbindungen I und II, in denen R¹ und R² nicht gleich Wasserstoff sind, wobei hier wiederum solche Verbindungen bevorzugt werden, in denen R¹ = R² ist.

In den neuen 2,5-disubstituierten 2-Formylcyclopentanone I haben die Reste R¹ und R² die gleiche Bedeutung.

Ausgangsstoffe der Formel II sind beispielsweise:
2,5-Dimethyl-2-formyl-3,4-dihydropyran
2,5-Diethyl-2-formyl-3,4-dihydropyran
2,5-Di-n-propyl-2-formyl-3,4-dihydropyran
2,5-Di-isopropyl-2-formyl-3,4-dihydropyran
2,5-Di-n-butyl-2-formyl-3,4-dihydropyran
2,5-Di(3-methoxicarbonyl-propyl)2-formyl-3,4-dihydropyran
2,5-Di(methoxicarbonylmethyl)-2-formyl-3,4-dihydropyran
2-Methyl-2-formyl-3,4-dihydropyran
5-Methyl-2-formyl-3,4-dihydropyran
2,5-Di(-2-propenyl)-2-formyl-3,4-dihydropyran.

Endprodukte der Formel I sind beispielsweise:
2,5-Dimethyl-2-formylcyclopentanon
2,5-Diethyl-2-formylcyclopentanon
2,5-Di-n-propyl-2-formylcyclopentanon
2,5-Di-isopropyl-2-formylcyclopentanon
2,5-Di-n-butyl-2-formylcyclopentanon
2,5-Di(3-methoxicarbonyl-propyl)-2-formylcyclopentanon
2,5-Di(methoxicarbonylmethyl)-2-formylcyclopentanon
2-Methyl-2-formyl-cyclopentanon
5-Methyl-2-formyl-cyclopentanon
2,5-Di-(2-propenyl)-2-formyl-cyclopentanon.

Neue 2-Formylcyclopentanone I sind:
2,5-Dimethyl-2-formylcyclopentanon
2,5-Diethyl-2-formylcyclopentanon
2,5-Di-n-propyl-2-formylcyclopentanon
2,5-Di-isopropyl-2-formylcyclopentanon
2,5-Di-n-butyl-2-formylcyclopentanon
2,5-Di-(3-methoxicarbonyl-propyl)-2-formylcyclopentanon
2,5-Di-(methoxicarbonylmethyl)-2-formylcyclopentanon

Die 2-Formylcyclopentanone I eignen sich z.B. als Lösungsmittel oder als Zwischenprodukte zur Synthese von Riechstoffen, Pharmazeutika und Kunststoffvorprodukten.

### b) Für den Fall, daß R³ für Wasserstoff steht, sind die Cyclopentanone I nach folgenden Methoden erhältlich:

b₁) Die Umsetzung erfolgt durch Kontakt von 2-Formyl-3,4-dihydropyranen II mit Wasser oder einem Alkohol an aciden Heterogenkatalysatoren nach folgender Reaktionsgleichung: 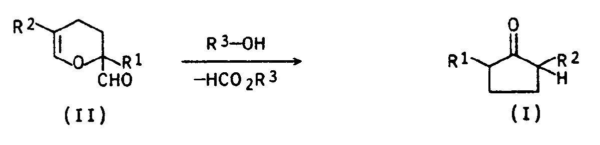
   Die Reaktion kann diskontinuierlich oder vorzugsweise kontinuierlich in der Gasphase bei 100 bis 500°C und 0,1 mbar bis 10 bar durchgeführt werden.
   Die Gasphasenreaktion kann beispielsweise bei Temperaturen von 100 bis 450°C, vorzugsweise bei 100 bis 350°C und Drücken von 0,1 mbar bis 5 bar, besonders bevorzugt bei 200 bis 300°C und Drücken von 0,5 mbar bis 2 bar durchgeführt werden. Bei der Umsetzung in der Gasphase hält man vorteilhaft eine Katalysatorbelastung von 0,01 bis 40, insbesondere von 0,05 bis 10 g Ausgangsstoff der Formel II je g Katalysator und Stunde ein (WHSV = g Einsatzgemisch/g Katalysator und Stunde). Die Gasphasenreaktion kann in einem Festbett oder in einem Wirbelbett ausgeführt werden.
   Das Verfahren wird im allgemeinen bei Normaldruck oder je nach Flüchtigkeit der Ausgangsverbindung bei vermindertem oder erhöhtem Druck vorzugsweise kontinuierlich durchgeführt.
   Das Molverhältnis von Wasser oder Alkohol zum 2-Formyl-3,4-dihydropyran II beträgt von 0,5 : 1 bis 20 : 1, bevorzugt 1 : 1 bis 10 : 1, besonders bevorzugt 2 : 1 bis 7 : 1.
   Als Alkohole eignen sich solche mit 1 bis 20 Kohlenstoffatomen, bevorzugt solche mit 1 bis 8 C-Atomen, besonders bevorzugt solche mit 1 bis 4 C-Atomen wie Methanol und Ethanol.
   Schwerflüchtige oder feste Ausgangsstoffe werden in gelöster Form z.B. in Tetrahydrofuran-, Toluol- oder Petrolether-Lösung eingesetzt. Allgemein ist auch eine Verdünnung mit diesen Lösungsmitteln oder mit Inertgasen wie N₂, Ar, möglich und zweckmäßig.
   Als Nebenprodukte können gegebenenfalls 2-Formyl-cyclopentanone der Formel I mit R³ = CHO gebildet werden, deren Anteil durch geeignete Maßnahmen wie Erhöhen der Temperatur oder des Wasser/Alkohol-Zusatzes bzw. Erniedrigen der Katalysatorbelastung fast vollständig zurückgedrängt werden kann.
   Nach der Umsetzung werden die entstandenen Produkte durch übliche Verfahren, z.B. durch Destillation aus dem Reaktionsgemisch isoliert; nichtumgesetzte Ausgangsstoffe werden gegebenenfalls in die Umsetzung zurückgeführt.
   Bevorzugt werden Reaktionsprodukte sofort in eine Trennung eingebracht und z.B. in einer Fraktionierkolonne in ihre Einzelkomponenten zerlegt.
   Als acide Heterogenkatalysatoren für das erfindungsgemäße Verfahren eignen sich saure Oxide, die im wesentlichen aus Oxiden der II. bis V. Hauptgruppe, der III. bis VII. Nebengruppe, aus Oxiden der Seltenen Erdmetalle oder aus einem Gemisch der genannten Oxide bestehen. So sind beispielsweise Boroxid, Aluminiumoxid, Siliciumoxid, z. B. in Form von Kieselgel, Kieselgur, Quarz oder auch Zinndioxid, Titandioxid, Ceroxid oder ein Gemisch derartiger Oxide geeignet. Zusätzlich können die Katalysatoren noch durch Aufbringen von Zusätzen, wie z. B. Phosphorsäure modifiziert werden. Weitere geeignete Katalysatoren sind Phosphate, wie Aluminium-oder Siliciumaluminiumphosphate. Bevorzugt sind Siliciumoxid enthaltende Katalysatoren. Ganz besonders bewährt hat sich der Einsatz von Silciumdioxid.
   Des weiteren eignen sich Zeolithe.
   Die Katalysatoren können wahlweise als 2- bis 4-mm-Stränge oder als Tabletten mit 3 bis 5 mm Durchmesser, als Splitt mit Teilchengrößen von 0,05 bis 1 mm, insbesondere 0,1 bis 0,5 mm, als Pulver mit Teilchengrößen von 0,1 bis 0,5 mm oder als Wirbelkontakt eingesetzt werden.
   Die Substituenten R¹ und R² in den Verbindungen I und II haben folgende Bedeutungen, wobei R¹ oder R² auch für Wasserstoff stehen kann:
   - unverzweigtes oder verzweigtes C₁- bis C₁₀-Alkyl, vorzugsweise C₁- bis C₈-Alkyl, besonders bevorzugt C₁- bis C₄-Alkyl wie Methyl, Ethyl, n-Propyl, iso-Propyl, n-Butyl, iso-Butyl, sec.-Butyl und tert.-Butyl,
   - unverzweigtes oder verzweigtes C₁- bis C₁₀-Alkyl, das durch cycloaliphatische Reste wie Cycloalkyl, aromatische Reste wie Aryl, besonders Phenyl oder heterocyclische Reste wie Pyridyl, einen Alkenyl- oder Alkinylrest oder durch Carbalkoxi-, Carboxi-, Alkylamino-, Acyl-, Phosphonester, Hydroxi-, Ether-, Cycloether-, Thioether-, Cyclothioethergruppen substituiert ist, bevorzugt mit C₃- bis C₈-Cycloalkyl, C₁- bis C₄-Carbalkoxi, Carboxi, C₂- bis C₄-Alkenyl, besonders bevorzugt C₅- bis C₆-Cycloalkyl und C₁- bis C₃-Carbalkoxi.

   Bevorzugt sind Verbindungen I und II, in denen R¹ und R² ungleich Wasserstoff sind, wobei hier wiederum solche Verbindungen besonders bevorzugt werden, in denen R¹ = R² ist.
   Ausgangsstoffe der Formel II sind beispielsweise:
   2,5-Dimethyl-2-formyl-3,4-dihydropyran
   2,5-Diethyl-2-formyl-3,4-dihydropyran
   2,5-Di-n-propyl-2-formyl-3,4-dihydropyran
   2,5-Di-isopropyl-2-formyl-3,4-dihydropyran
   2,5-Di-n-butyl-2-formyl-3,4-dihydropyran
   2,5-Di(3-methoxicarbonyl-propyl)-2-formyl-3,4-dihydropyran
   2,5-Di(methoxicarbonylmethyl)-2-formyl-3,4-dihydropyran
   2-Methyl-2-formyl-3,4-dihydropyran
   5-Methyl-2-formyl-3,4-dihydropyran
   2,5-Di-(-2-propenyl)-2-formyl-3,4-dihydropyran
   Endprodukte der Formel I sind beispielsweise:
   2,5-Dimethylcyclopentanon
   2,5-Diethylcyclopentanon
   2,5-Di-n-propylcyclopentanon
   2,5-Di-isopropylcyclopentanon
   2,5-Di-n-butylcyclopentanon
   2,5-Di-(3-methoxicarbonyl-propyl)-cyclopentanon
   2,5-Di-(methoxicarbonylmethyl)-cyclopentanon
   2-Methylcyclopentanon
   2,5-Di-(-2-propenyl)-cyclopentanon
b₂) Die Umsetzung der 2-Formyl-3,4-dihydropyrane oder der Acroleine mit Wasser oder mit Wasser in Gegenwart einer Säure oder mit einem Alkohol in Gegenwart einer Säure kann diskontinuierlich oder vorzugsweise kontinuierlich in der Flüssigphase bei 150 bis 400°C, vorzugsweise bei 200 bis 300°C und Drücken von 1 bis 200 bar, besonders bevorzugt bei 230 bis 280°C und Drücken von 5 bis 150 bar durchgeführt werden; der angewendete Druck kann auch mehr als 200 bar betragen; ggf. wird die Reaktion auch bei Unterdruck durchgeführt.

Das erfindungsgemäße Verfahren wird im allgemeinen unter Druck diskontinuierlich z.B. im Autoklaven oder vorzugsweise kontinuierlich z.B. im Rohrreaktor bei Verweilzeiten von 10 Sekunden bis 3 Stunden vorzugsweise von 1 Minute bis 2 Stunden durchgeführt.

Zur kontinuierlichen Durchführung des erfindungsgemäßen Verfahrens leitet man zweckmäßig die Verbindungen II oder III und Wasser mit oder ohne Säure oder einen Alkohol mit Säure getrennt über separate Zuleitungen, gegebenenfalls unter Mitverwendung eines inerten Lösungsmittels in den auf Reaktionstemperatur erhitzten Reaktor. Das Reaktionsgemisch wird unter erhöhtem Druck durch den Reaktor gepumpt, anschließend entspannt und abgekühlt und dann nach üblichen Verfahren wie Phasentrennung, Extraktion und Destillation aufgearbeitet.

Bei der diskontinuierlichen Durchführung werden die Verbindungen II oder III mit Wasser und ggf. einer Säure oder einem Alkohol und einer Säure gegebenenfalls unter Mitverwendung eines inerten Lösungsmittels z.B. im geschlossenen Autoklav auf die Reaktionstemperatur erhitzt und anschließend wie üblich aufgearbeitet.

Je Mol der Verbindungen II bzw. III setzt man 0,1 bis 50 mol, bevorzugt 0,5 bis 20 mol und besonders bevorzugt 1 bis 10 mol Wasser oder einen Alkohol ein.

Als Alkohole eignen sich solche mit 1 bis 20 Kohlenstoffatomen, bevorzugt solche mit 1 bis 8 C-Atomen, besonders bevorzugt solche mit 1 bis 4 C-Atomen wie Methanol, Ethanol und n-Propanol.

Als saure Katalysatoren eignen sich bevorzugt Homogenkatalysatoren wie anorganische Säuren wie Schwefelsäure, Salzsäure und Phosphorsäure oder organische Säuren. Besonders bevorzugt sind organische Säuren wie Carbonsäuren z.B. Ameisensäure, Essigsäure und Propionsäure oder Sulfonsäuren z.B. Methansulfonsäure und p-Toluolsulfonsäure.

Bei Einsatz von Wasser können auch Heterogenkatalysatoren wie z.B. Siliciumdioxid verwendet werden.

Bei Einsatz eines Alkohols beträgt der Säurezusatz 0,001 bis 10 mol%, bezogen auf Verbindungen II oder III, bevorzugt 0,01 bis 1 mol%, besonders bevorzugt 0,1 bis 0,5 mol%. Bei Einsatz von Wasser kann auf die Säurezugabe verzichtet werden; ggf. erreicht man hier durch Zugabe von 0,01 bis 1 mol% Säure eine höhere Reaktionsgeschwindigkeit und eine leicht erhöhte Ausbeute.

Die Substituenten R¹ und R² in den Verbindungen I und II haben folgende Bedeutungen, wobei R¹ oder R² auch für Wasserstoff stehen kann:
- unverzweigtes oder verzweigtes C₁- bis C₁₀-Alkyl, vorzugsweise C₁- bis C₈-Alkyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- unverzweigtes oder verzweigtes C₁- bis C₁₀-Alkyl, das durch cycloaliphatische Reste wie Cycloalkyl, aromatische Reste wie Aryl, besonders Phenyl oder heterocyclische Reste wie Pyridyl, einen Alkenyl- oder Alkinylrest oder durch Carbalkoxi-, Carboxi-, Alkylamino-, Acyl-, Phosphonester-, Hydroxi-, Ether-, Cycloether-, Thioether-, Cyclothioethergruppen substituiert ist, bevorzugt C₃- bis C₈-Cycloalkyl, C₁-bis C₄-Carbalkoxi, Carboxi, C₂- bis C₄-Alkenyl, besonders bevorzugt C₅-bis C₆-Cycloalkyl.

Bevorzugt sind Verbindungen I und II, in denen R¹ und R² ungleich Wasserstoff sind, wobei hier wiederum solche Verbindungen besonders bevorzugt werden, in denen R¹ = R² ist.

Ausgangsstoffe der Formel II sind beispielsweise:
2,5-Dimethyl-2-formyl-3,4-dihydropyran
2,5-Diethyl-2-formyl-3,4-dihydropyran
2,5-Di-n-propyl-2-formyl-3,4-dihydropyran
2,5-Di-isopropyl-2-formyl-3,4-dihydropyran
2,5-Di-n-butyl-2-formyl-3,4-dihydropyran
2,5-Di-(3-methoxicarbonyl-propyl)-2-formyl-3,4-dihydropyran
2,5-Di-(methoxicarbonylmethyl)-2-formyl-3,4-dihydropyran
2-Methyl-2-formyl-3,4-dihydropyran
5-Methyl-2-formyl-3,4-dihydropyran
2,5-Di-(2-propenyl)-2-formyl-3,4-dihydropyran

Ausgangsstoffe der Formel III sind beispielsweise:
Methacrolein
Ethylacrolein
n-Propylacrolein
Isopropylacrolein
n-Butylacrolein
(3-Methoxicarbonylpropyl)-acrolein
Methoxicarbonylmethyl-acrolein
(2-Propenyl)-acrolein

Endprodukte der Formel I des erfindungsgemäßen Verfahrens sind:
2,5-Dimethylcyclopentanon
2,5-Diethylcyclopentanon
2,5-Di-n-propylcyclopentanon
2,5-Di-isopropylcyclopentanon
2,5-Di-n-butylcyclopentanon
2,5-Di(3-methoxicarbonyl-propyl)-cyclopentanon
2,5-Di(methoxicarbonylmethyl)-cyclopentanon
2-Methylcyclopentanon
2,5-Di-(2-propenyl)-cyclopentanon.

Die Cyclopentanone I eignen sich z.B. als Lösungsmittel (US-A-2,875,249) oder als Zwischenprodukte zur Synthese von Herbiziden (US-A-4,155,744), Süßstoffen (US-A-4,692,513; EP-A-34,876) und Pharmazeutika (DE-A-2,344,527; DE-A-1,670,753).

### Beispiele

### A: Herstellung von 2-Formyl-2,5-dimethylcyclopentanon aus 2-Formyl-2,5-dimethyl-3,4-dihydropyran (Dimer-Methacrolein)

### Beispiel 1

Pro Stunde wurden 60,4 g Dimer-Methacrolein bei Normaldruck verdampft und zusammen mit 50 l Stickstoff in einen auf 200 °C beheizten Reaktor (Quarzrohr-Innendurchmesser 30 mm) eingeleitet, der mit 200 ml SiO₂-Strängen (3 mm Durchmesser, Schüttgewicht 427 g/l) gefüllt war. Die entstandenen Reaktionsdämpfe kondensierte man. Nach 13-stündigem Betrieb wurden 774,4 g Reaktionsgemisch der folgenden Zusammensetzung (Quant. GC-Analyse) erhalten:
93,6 Gew.-% 2-Formyl-2,5-dimethylcyclopentanon
2,0 Gew.-% 2,5-Dimethylcyclopentanon
1,1 Gew.-% Dimer-Methacrolein

Dies entspricht einer Formylketon-Selektivität von 93,3 % bei 98,9 % Umsatz. Fraktionierte Destillation lieferte ein cis/trans-Gemisch des Formylketons als farblose Flüssigkeit (Sdp. 96 bis 98 °C/50 mbar).

### Beispiel 2

Aus einem Verdampfer wurden bei Normaldruck pro Stunde 51,4 g Dimer-Methacrolein mit 50 l Stickstoff in einen auf 300°C beheizten Reaktor (Quarzrohr-Innendurchmesser 30 mm) eingeleitet, der mit 200 ml Katalysator, bestehend aus 20 Gew.-% B2O3 und 80 Gew.-% SiO2, gepreßt als 3-mm-Stränge und einem Schüttgewicht von 624 g je Liter, gefüllt war. Die entstandenen Reaktionsdämpfe kondensierte man. Nach 2,5stündigem Betrieb wurden 122,5 g Reaktionsgemisch der folgenden Zusammensetzung erhalten (quant. GC-Analyse):
5,0 Gew.-% Dimer-Methacrolein
83,5 Gew.-% 2-Formyl-2,5-dimethyl-cyclopentanon
3,6 Gew.-% 2,5-Dimethylcyclopentanon

Dies entspricht einem Umsatz von 95,2 % und einer Formylketon-Selektivität von 83,7 %.

### Beispiel 3

Der mit 7,0 ml SiO₂-Strängen (3 mm Durchmesser, Schüttgewicht 427 g/l) gefüllte Teil eines V2A-Stahlrohres (6 mm Innendurchmesser) wird im Ölbad auf 180°C beheizt. Unter einem Druck von 50 bar leitet man pro Stunde eine Lösung von 50,6 g Dimer-Methacrolein in 59,4 g Cyclohexan über den Katalysator. Nach Entspannen und Abkühlen fallen stündlich 110,0 g Reaktionsaustragan, der neben Cyclohexan 31.6 g 2-Formyl-2,5-dimethylcyclopentanon und 16,5 g Dimer-Methacrolein enthält (Quant. GC-Analyse). Dies entspricht einer Selektivität von 92,7 % bei 67,4 % Umsatz.

### B: Herstellung von 2-Formyl-2,5-diethyl-cyclopentanon aus 2-Formyl-2,5-diethyl-3,4-dihydropyran (Dimer-Ethylacrolein)

### Beispiel 4

49,3 g Dimer-Ethylacrolein wurden pro Stunde bei Atmosphärendruck verdampft und mit 50 Liter Stickstoff/h in einen auf 200°C temperierten Reaktor (Quarzrohrinnendurchmesser 30 mm) eingeleitet, der mit 200 ml SiO2-Strängen vom Durchmesser 3 mm und einer Schüttdichte von 427 g je Liter gefüllt war. Durch Kondensation der Reaktiondämpfe erhielt man nach 16,5 Stunden 808,5 g Reaktionsaustrag, der sich wie folgt zusammensetzte (quant. GC-Analyse):
4,0 Gew.-% 2-Formyl-2,5-diethyl-3,4-dihydropyran
94,2 Gew.-% 2-Formyl-2,5-diethylcyclopentanon
2,0 Gew.-% 2,5-Diethylcyclpentanon

Dies entspricht einer Formylketon-Selektivität von 95,4 % bei einem Umsatz von 96,0 %.

Fraktionierte Destillation des Rohproduktes führte zu reinem 2-Formyl-2,5-diethyl-cyclopentanon (cis/trans Gemisch), einer farblosen Flüssigkeit vom Siedepunkt: 128 bis 130°C/50 mbar.

### C: Herstellung von 2-Formyl-2,5-diisopropylcyclopentanon aus 2-Formyl-2,5-diisopropyl-3,4-dihydropyran (Dimer-Isopropylacrolein)

### Beispiel 5

Aus einem Verdampfer wurden bei Normaldruck pro Stunde je 49,3 g Dimer-Isopropylacrolein und 50 Liter Stickstoff in einen auf 200°C beheizten Reaktor (Quarzrohrinnendurchmesser 30 mm) eingeleitet, der mit 200 ml SiO₂-Strängen (Durchmesser: 3 mm; Schüttdichte: 427 g/l) gefüllt war. Die entstandenen Reaktionsdämpfe kondensierte man. Nach 4-stündigem Betrieb wurden 193,5 g Reaktionsaustrag erhalten, der gemäß quantitativer GC-Analytik 94,8 Gew.-% 2-Formyl-2,5-diisopropylcyclopentanon und 4,3 Gew.-% 2,5-Diisopropylcyclopentanon enthielt. Bei vollständigem Umsatz an Dimer-Isopropylacrolein entspricht dies einer Ausbeute an Formylketon von 93,0 %. Fraktionierte Destillation führte zu reinem 2-Formyl-2,5-diisopopylcyclopentanon (cis/trans-Gemisch, Sdp. 142 bis 143°C/20 mbar); Nebenprodukt: 2,5-Diisopropylcyclopentanon: Sdp. 128 bis 133 °C/50 mbar.

### D: Herstellung von 2,5-Dimethylcyclopentanon aus 2-Formyl-2,5-dimethyl-3,4-dihydropyran (Dimer-Methacrolein)

### Beispiel 6

Pro Stunde wurden eine Lösung aus 23,0 g Dimer-Methacrolein und 26,3 g Methanol bei Normaldruck verdampft und zusammen mit 25 l Stickstoff in einen auf 250 °C beheizten Reaktor (Quarzrohr-Innendurchmesser 30 mm) eingeleitet, der mit 200 ml SiO₂-Strängen (3 mm Durchmesser, Schüttgewicht 427 g/l) gefüllt war. Die entstandenen Reaktionsdämpfe kondensierte man. Nach 2-stündigem Betrieb wurden 86,3 g Reaktionsgemisch mit einem durch quantitative Gaschromatographie ermittelten Anteil von 38,6 Gew.-% 2,5-Dimethylcyclopentanon erhalten. Dies entspricht einer Ausbeute von 90,5 % bei vollständigem Umsatz. Durch fraktionierende Destillation erhielt man neben Methanol und Methylformiat reines 2,5-Dimethylcyclopentanon (cis/trans-Gemisch, Sdp. 76 bis 86 °C/100 mbar).

### E: Herstellung von 2,5-Diethyl-cyclopentanon aus 2-Formyl-2,5-diethyl-3,4-dihydropyran (Dimer-Ethylacrolein)

### Beispiel 7

Eine Lösung aus 24,2 g Dimer-Ethylacrolein und 23,0 g Methanol wurden pro Stunde bei Atmosphärendruck verdampft und mit 25 Liter Stickstoff/h in einen auf 300 °C temperierten Reaktor (Quarzrohrinnendurchmesser 30 mm) eingeleitet, der mit 200 ml SiO₂-Strängen (Durchmesser 3 mm, Schüttdichte 427 g/l) gefüllt war. Durch Kondensation der Reaktionsdämpfe erhielt man nach 3 Stunden 129,5 g Reaktionsaustrag mit einem Anteil von 44,8 Gew.-% 2,5-Diethylcyclopentanon (quant. Gaschromatographie). Dies entspricht einer Ausbeute von 96,9 % bei quantitativem Umsatz. Fraktionierende Destillation des Rohproduktes lieferte neben Methanol und Methylformiat reines 2,5-Diethyl-cyclopentanon (cis/trans Gemisch) als farblose Flüssigkeit vom Siedepunkt 54 bis 57 °C/10 mbar.

### F: Herstellung von 2,5-Di-n-propylcyclopentanon aus 2-Formyl-2,5-di-n-propyl-3,4-dihydropyran (Dimer-n-Propylacrolein)

### Beispiel 8

In einen auf 250 °C temperierten Reaktor (Quarzrohr-Innendurchmesser 60 mm), gefüllt mit 300 ml SiO₂-Splitt (0,1 bis 0,3 mm, Schüttgewicht 363 g/l), wurden von unten 50 l/h Wirbel-Stickstoff zugeführt. Über einen Zeitraum von 8 Stunden dosierte man eine Lösung aus 254,4 g Dimer-n-Propylacrolein und 207,2 g Methanol durch eine Metall-Kappillare direkt in das Wirbelbett (in 1 cm Abstand vom unteren Ende der Katalysator-Schüttung). Kondensation der Reaktionsdämpfe ergab 419,4 g Reaktionsaustrag, der 174,1 g 2,5-Di-n-propyl-cyclopentanon enthielt. Bei vollständigem Umsatz an Dimer-Propylacrolein entspricht dies einer Ausbeute von 79,8 %. Fraktionierende Destillation führte zu reinem 2,5-Di-n-propylcyclopentanon (cis/trans-Gemisch, Sdp. 125 bis 128 °C/50 mbar).

### Vergleichsbeispiele:

### Beispiel 9 analog US-A-3,812,190, Beispiel 9

Pro Stunde wurden 25,4 g Dimer-Methacrolein bei Normaldruck verdampft und zusammen mit 25 l Stickstoff in einen auf 225 °C beheizten Reaktor (Quarzrohr-Innendurchmesser 27 mm) eingeleitet, der mit 100 ml Katalysator, bestehend aus 0,72 Gew.-% Pd und 99,28 Gew.-% gamma-Al₂O₃, gepreßt als 4-mm-Stränge und einem Schüttgewicht von 690 g/l, gefüllt war. Die entstandenen Reaktionsdämpfe kondensierte man. Nach 3stündigem Betrieb wurden 47,7 g Reaktionsgemisch mit einem durch quantitative Gaschromatographie ermittelten Anteil von 21,0 Gew.-% 2,5-Dimethylcyclopentanon erhalten. Dies entspricht einer Ausbeute von 16,4 % bei vollständigem Umsatz.

### Beispiel 10 analog US-A-3,812,190, Beispiel 7

Aus einem Verdampfer wurden bei Normaldruck pro Stunde 25,4 g Dimer-Methacrolein mit 25 l Stickstoff in einen auf 225 °C beheizten Reaktor (Quarzrohr-Innendurchmesser 27 mm) eingeleitet, der mit 100 ml NiO-Tabletten (3 x 3 mm, Schüttgewicht 2330 g/l) gefüllt war. Die entstandenen Reaktionsdämpfe kondensierte man. Nach 3stündigem Betrieb wurden 69,8 g Reaktionsgemisch erhalten, welches gemäß quant. Gaschromatographie 92,4 Gew.-% Dimer-Methacrolein und 0,6 Gew.-% 2,5-Dimethylcyclopentanon enthielt. Dies entspricht einem Umsatz von 15,4 % und einer Selektivität von 4,5 %.

### G: Herstellung von 2,5-Dimethylcyclopentanon aus 2,5-Dimethyl-2-formyl-3,4-dihydropyran (Dimer-Methacroleinen)

### Beispiel 11

Durch eine Rohrschlange von 10 m Länge und einem Innendurchmesser von 2,17 mm wurden bei 100 bar und 250°C pro Stunde 40,9 g Dimer-Methacrolein sowie eine Lösung von 110 mg p-Toluolsulfonsäure in 10,8 g Wasser gepumpt. Am Reaktorausgang wurde das Reaktionsgemisch auf Normaldruck entspannt und abgekühlt. Nach Abtrennen der wäßrigen Phase verblieben pro Stunde 43,2 g organische Phase, die gemäß quantitativer Gaschromatographie 57,4 Gew.-% 2,5-Dimethylcyclopentanon enthielten. Bei vollständigem Umsatz des Dimer-Methacroleins entspricht dies einer Ausbeute von 75,8%. Nach 5-stündigem Betrieb wurde die organische Phase mit Natronlauge neutral gewaschen und fraktioniert destilliert; man erhielt 121,5 g 2,5-Dimethylcyclopentanon (cis/trans-Gemisch, Siedep. 76 bis 86°C/100 mbar).

### Beispiel 12

Analog Beispiel 11 wurden bei 275°C pro Stunde 482,4 g Dimer-Methacrolein sowie eine Lösung von 1,20 g p-Toluolsulfonsäure in 123,8 g Wasser durch die Rohrschlange gepumpt. Gemäß quantitativer Gaschromatographie der org. Phase des Reaktionsaustrages fielen pro Stunde 279,2 g 2,5-Dimethylcyclopentanon an, entsprechend einer Ausbeute von 72,3%.

### Beispiel 13

Wie in Beispiel 11 beschrieben wurden bei 275 °C stündlich 52,4 g Dimer-Methacrolein und eine Lösung aus 70 mg p-Toluolsulfonsäure in 29,6 g Methanol durch die Rohrschlange gepumpt. Quantitative gaschromatographische Analyse des Reaktionsaustrages ergab eine Ausbeute von 25,4 g 2,5-Dimethylcyclopentanon pro Stunde, entsprechend einer Ausbeute von 60,6 %.

### Beispiel 14

28,0 g Dimer-Methacrolein wurden zusammen mit 31 g Wasser unter Rühren in einem verschlossenem Autoklaven (0,3 Liter) innerhalb von 4 Stunden auf 250°C erhitzt und 2 Stunden bei dieser Temperatur belassen. Nach Abkühlen des Reaktionsgemisches verbleiben 26,8 g org. Phase, die 14,6 g 2,5-Dimethylcyclopentanon enthielten; die Ausbeute betrug 65,2 %.

### H: Herstellung von 2,5-Dimethylcyclopentanon aus Methacrolein

### Beispiel 15

Analog Beispiel 11 wurden bei 250°C durch die Rohrschlange pro Stunde 18,2 g Methacrolein und 4,7 g Wasser gepumpt. Nach Abtrennen der wäßrigen Phase verbleiben stündlich 18,6 g org. Phase mit einem gaschromatographisch bestimmmten Anteil an 2,5-Dimethylcyclopentanon von 48,3 %, entsprechend einer Ausbeute von 61,7 %.

### Beispiel 16

910 g Methacrolein und 234 g Wasser wurden in einem Autoklav (2,5 Liter) unter Rühren innerhalb von 3 Stunden auf 250°C erhitzt und 2 Stunden bei dieser Temperatur belassen. Nach Abkühlen wurde die wäßrige Phase abgetrennt, zweimal mit je 50 ml Methyl-tert.-butylether extrahiert. Fraktionierte Destillation der vereinigten organischen Phasen lieferte 398,2 g 2,5-Dimethylcyclopentanon, entsprechend einer Ausbeute von 54,7 %.

### I: Herstellung von 2,5-Diethylcyclopentanon aus 2,5-Diethyl-2-formyl-3,4-dihydropyran (Dimer-Ethylacrolein)

### Beispiel 17

Analog Beispiel 11 wurden bei 275°C stündlich 88,7 g Dimer-Ethylacrolein mit einer Lösung aus 100 mg p-Toluolsulfonsäure in 21,3 g Wasser durch die Rohrschlange gepumpt. Pro Stunde fielen 84,0 g org. Phase an, die gemäß quantitativer Gaschromatographie zu 51,6 Gew.-% 2,5-Diethylcyclopentanon enthielten, entsprechend einer Ausbeute von 58,6 %. Fraktionierte Destillation lieferte reines 2,5-Diethylcyclopentanon im cis/trans Gemisch (Sdp. 54 bis 57°C/10 mbar).

### J: Herstellung von 2,5-Diisopropylcyclopentanon aus 2,5-Diisolpropyl-2-formyl-3,4-dihydropyran (Dimer-Isopropylacrolein)

### Beispiel 18

Wie in Beispiel 11 wurden durch die auf 285°C temperierte Rohrschlange pro Stunde 99,2 g Dimer-Isopropylacrolein sowie eine Lösung aus 500 mg p-Toluolsulfonsäure in 19,7 g Wasser gepumpt. Ein quantitatives Gaschromatogramm der anfallenden org. Phase ergab eine Ausbeute von stündlich 46,9 g 2,5-Diisopropylcyclopentanon (55,2 % der Th.), welches nach fraktionierter Destillation als farblose Flüssigkeit vom Siedepunkt 128 bis 133°C/50 mbar erhalten wurde.

## Patentansprüche

1. Verfahren zur Herstellung von Cyclopentanonen der allgemeinen Formel I 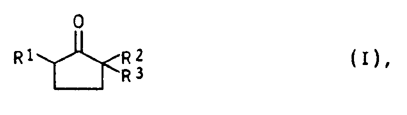 in der R¹ und R² einen organischen Rest oder R¹ oder R² gegebenenfalls Wasserstoff bedeuten und R³ für Wasserstoff oder Formylgruppe steht, dadurch gekennzeichnet, daß man 2-Formyl-3,4-dihydropyrane der allgemeinen Formel II 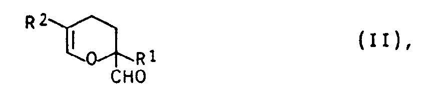 in der R¹ und R² die oben genannte Bedeutung haben,
a) für den Fall, daß R³ für eine Formylgruppe steht, die Umsetzung in Gegenwart eines aciden Heterogenkatalysators bei Temperaturen von 50 bis 500°C,
b) für den Fall, daß R³ für Wasserstoff steht,
b₁) die Umsetzung mit Wasser oder einem Alkohol in Gegenwart eines aciden Heterogenkatalysators bei Temepraturen von 100 bis 500°C in der Gasphase oder
b₂) die Umsetzung der Verbindungen II oder Acroleine der allgemeinen Formel III 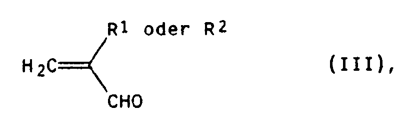 mit Wasser oder mit Wasser in Gegenwart einer Säure oder mit einem Alkohol in Gegenwart einer Säure bei Temperaturen von 150 bis 400°C in der Flüssigphase durchführt.

2. Verfahren nach Anspruch 1, dadurch gekennzeichnet, daß man als acide Heterogenkatalysatoren saure Oxide oder Phosphate verwendet.

3. Verfahren nach Anspruch 1, dadurch gekennzeichnet, daß man als aciden Heterogenkatalysator Siliciumdioxid verwendet.

4. Verfahren nach Anspruch 1, dadurch gekennzeichnet, daß man als Säuren anorganische und organische Säuren verwendet.

5. Verfahren nach Anspruch 1, dadurch gekennzeichnet, daß man als organische Säuren Carbon- und Sulfonsäuren verwendet.

6. Verfahren nach Anspruch 1, dadurch gekennzeichnet, daß die Reste R¹ und R² C₁- bis C₁₀-Alkyl bedeuten, das gegebenenfalls durch cycloaliphatische, aromatische oder heterocyclische Reste, einen Alkenyl- oder Alkinylrest oder durch Carbalkoxi-, Carboxi-, Alkylamino-, Acyl-, Hydroxi-, Ether-, Thioethergruppen substituiert ist.

7. Verfahren nach Anspruch 1, dadurch gekennzeichnet, daß die Reste R¹ und R²in den Verbindungen die gleiche Bedeutung haben.

8. Verfahren nach Anspruch 1, dadurch gekennzeichnet, daß man Wasser oder einen Alkohol zum 2-Formyl-3,4-dihydropyran II im Molverhältnis von 0,5:1 bis 20:1 verwendet.

9. Verfahren nach Anspruch 1, dadurch gekennzeichnet, daß man Wasser oder einen Alkohol zum 2-Formyl-3,4-dihydropyran II im Molverhältnis von 1:1 bis 10:1 verwendet.

10. 2,5-Disubstituierte 2-Formylcyclopentanone der allgemeinen Formel I nach Anspruch 1, in der R¹ und R² gleich sind und für C₁- bis C₁₀-Alkyl stehen, das gegebenenfalls durch cycloaliphatische, aromatische oder heterocyclische Reste, einen Alkenyl- oder Alkinylrest oder durch Carbalkoxi-, Carboxi-, Alkylamino-, Acyl-, Hydroxi-, Ether-, Thioethergruppen substituiert ist.

## Claims

1. A process for the preparation of a cyclopentanone of the general formula I 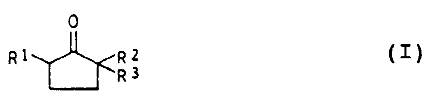 where R¹ and R² are each an organic radical or R¹ or R² may be hydrogen and R³ is hydrogen or formyl, wherein a 2-formyl-3,4-dihydropyran of the general formula II 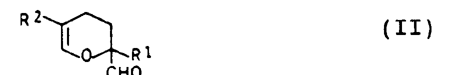 where R¹ and R² have the abovementioned meanings,
a) where R³ is formyl, is converted in the presence of an acidic heterogeneous catalyst at from 50 to 500°C, and
b) where R³ is hydrogen,
b₁) is reacted with water or an alcohol in the presence of an acidic heterogeneous catalyst at from 100 to 500°C in the gas phase or
b₂) a compound II or an acrolein of the general formula III 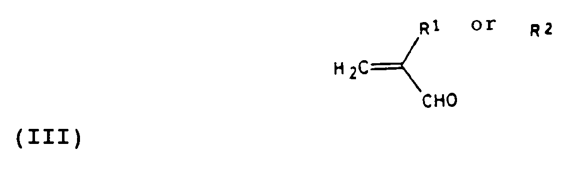 is reacted with water or with water in the presence of an acid or with an alcohol in the presence of an acid at from 150 to 400°C in the liquid phase.

2. A process as claimed in claim 1, wherein the acidic heterogeneous catalyst used is an acid oxide or phosphate.

3. A process as claimed in claim 1, wherein the acidic heterogeneous catalyst used is silica.

4. A process as claimed in claim 1, wherein the acids used are inorganic and organic acids.

5. A process as claimed in claim 1, wherein the organic acids used are carboxylic and sulfonic acids.

6. A process as claimed in claim 1, wherein R¹ and R² are each C₁-C₁₀-alkyl which is unsubstituted or substituted by cycloaliphatic, aromatic or heterocyclic radicals, by an alkenyl or alkynyl radical or by carbalkoxy, carboxyl, alkylamino, acyl, hydroxyl, ether or thioether groups.

7. A process as claimed in claim 1, wherein R¹ and R² in the compounds have the same meaning.

8. A process as claimed in claim 1, wherein the molar ratio of water or of an alcohol to the 2-formyl-3,4-dihydropyran II is from 0.5 : 1 to 20 : 1.

9. A process as claimed in claim 1, wherein the molar ratio of water or of an alcohol to the 2-formyl-3,4-dihydropyran II is from 1 : 1 to 10 : 1.

10. A 2,5-disubstituted 2-formylcyclopentanone of the general formula I as claimed in claim 1, wherein R¹ and R² are identical and are each C₁-C₁₀-alkyl which is unsubstituted or substituted by cycloaliphatic, aromatic or heterocyclic radicals, by an alkenyl or alkynyl radical or by carbalkoxy, carboxyl, alkylamino, acyl, hydroxyl, ether or thioether groups.

## Revendications

1. Procédé de préparation de cyclopentanones de formule générale I 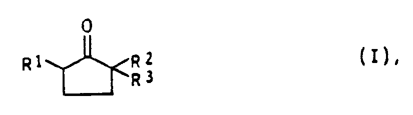 dans laquelle R¹ et R² représentent un reste organique ou R¹ ou R² représente éventuellement un atome d'hydrogène, et R³ est mis pour un atome d'hydrogène ou un groupe formyle, caractérisé en ce que l'on fait réagir du 2-formyl-3, 4-dihydropyranne de formule générale II 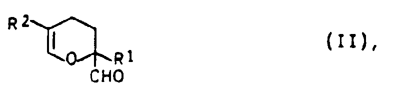 dans laquelle R¹ et R² ont les significations indiquées ci-dessus,
a) dans le cas où R³ est un groupe formyle, en présence d'un catalyseur hétérogène acide à des températures de 50 à 500°C,
b) dans le cas où R³ est un atome d'hydrogène,
b₁) avec de l'eau ou un alcool en présence d'un catalyseur hétérogène acide à des températures de 100 à 500°C en phase gazeuse ou
b₂) en ce que l'on fait réagir les composés II ou les acroléines de formule générale III 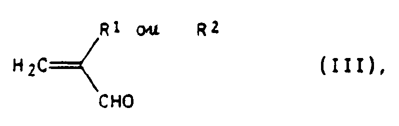 avec de l'eau ou avec de l'eau en présence d'un acide ou avec un alcool en présence d'un acide à des températures de 150 à 400°C en phase liquide.

2. Procédé selon la revendication 1, caractérisé en ce que l'on utilise comme catalyseurs hétérogènes acides des oxydes ou phosphates acides.

3. Procédé selon la revendication 1, caractérisé en ce que l'on utilise comme catalyseur hétérogène acides du dioxyde de silicium.

4. Procédé selon la revendication 1, caractérisé en ce que l'on utilise en tant qu'acides des acides inorganiques et organiques.

5. Procédé selon la revendication 1, caractérisé en ce que l'on utilise en tant qu'acides organiques des acides carboxyliques et sulfoniques.

6. Procédé selon la revendication 1, caractérisé en ce que les restes R¹ et R² représentent des groupes alkyle en C₁ à C₁₀, qui sont éventuellement substitués par des restes cycloaliphatiques, aromatiques ou hétérocycliques, par un reste alcényle ou alcinyle ou par des groupes alcoxycarbonyle, carboxy, alkylamino, acyle, hydroxy, éther, thioéther.

7. Procédé selon la revendication 1, caractérisé en ce que les restes R¹ et R² dans les composés ont la même signification.

8. Procédé selon la revendication 1, caractérisé en ce que l'on utilise de l'eau ou un alcool dans un rapport molaire de 0,5:1 à 20:1 par rapport au 2-formyl-3,4-dihydropyranne II.

9. Procédé selon la revendication 1, caractérisé en ce que l'on utilise de l'eau ou un alcool dans un rapport molaire de 1:1 à 10:1 par rapport au 2-formyl-3,4-dihydropyranne II.

10. 2-Formylcyclopentanones 2,5-disubstituées de formule générale I selon la revendication 1, dans laquelle R¹ et R² sont égaux et sont mis pour un groupe alkyle en C₁ à C₁₀, qui est éventuellement substitué par des restes cycloaliphatiques, aromatiques, ou hétérocycliques, par un reste alcényle ou alcinyle ou par des groupes alcoxyrarbonyle, carboxy, alkylamino, acyle, hydroxy, éther, thioéther.
